# EUROPEAN PATENT APPLICATION

(11) **EP 4 374 862 A1**
(43) Date of publication of application: **29.05.2024**
(21) Application number: 22846065.5
(22) Date of filing: 27.06.2022
(51) Int. Cl.: A61K 31/7048, A61K 36/65, A61P 21/00, A23L 33/125

(54) **COMPOSITION INCLUDING PAEONIFLORIN FOR PREVENTION OR TREATMENT OF CACHEXIA AND MUSCLE LOSS**

(30) Priority: 21.07.2021 KR 20210095733
(71) Applicant: Chung Ang University Industry Academic Cooperation Foundation, Seoul 06974 (KR)
(72) Inventor: YOON, Yoo Sik, Seoul 06270 (KR); SIM, In Ae, Goyang-si Gyeonggi-do 10468 (KR); LEE, Jong Kyu, Bucheon-si Gyeonggi-do 14618 (KR); LIM, Hye Mi, Yongin-si Gyeonggi-do 17045 (KR)
(74) Representative: J A Kemp LLP
(86) International application number: PCT/KR2022/009127
(87) International publication number: WO 2023/003193

(57) **Abstract**

The present invention relates to a pharmaceutical composition for preventing or treating cachexia and muscle loss containing paeoniflorin. The pharmaceutical composition containing paeoniflorin may be used to effectively prevent or treat cachexia and muscle loss.

## Description

### Technical Field

The present invention relates to a composition comprising paeoniflorin.

### Background Art

Cachexia is a syndrome that is commonly associated with chronic diseases such as cancer, tuberculosis, AIDS, and chronic obstructive pulmonary disease, and refers to a catabolic state of body metabolism characterized by persistent loss of appetite and weight loss, resulting in malnutrition, metabolic imbalance, and loss of muscle or fat. Cancer patients are characterized not only by cachexia, but also by side effects of various anticancer therapies used for cancer treatment, unlike patients with other chronic diseases (Fearon K. et al., Lancet Oncol 2011;12(5) :489-495) .

Cachexia occurs in 50 to 80% of gastrointestinal cancer patients and lung cancer patients, and the mortality caused by cachexia reaches 20 to 30%. Cachexia that occurs in cancer patients is characterized by weight loss due to muscle loss resulting from inflammatory responses caused by various cytokines and increased catabolism caused by metabolic changes. These changes lower the rate of response to anticancer chemotherapy or radiotherapy, make it difficult to achieve effective anticancer therapy, and lower the patient's quality of life.

It is known that mechanisms of cachexia include changes in the activity of the neuroendocrine system, secretion of various pro-inflammatory cytokines (TNF-α, IL-6, and IL-1β), GDF-15, and cancer-specific cachectic factors, the reduction in food intake by these mediators, and changes in metabolism. Cytokines are proteins that are secreted directly from tumors or produced by human immune cells in response to tumors, and they play an important role in immune control, and play the most important role in the development of cachexia by causing inflammation, loss of appetite, muscle loss, and weight loss (Argiles JM et al., Curr. Opin. Clin. Nutr. Metab. Care 1998;1: 245-251). Pro-inflammatory cytokines, such as TNF-α, IL-6, and IL-1β, are known to cause loss of appetite and weight loss by causing changes in brain appetite control center or gastrointestinal function (Durham WJ et al., Curr Opin Clin Nutr Metab Care. 2009; 12:72-77).

In addition, it has recently been found that GDF-15 (growth differentiation factor 15), a type of cytokine, acts as the cause of loss of appetite and weight loss by tumors or chemotherapy, and inhibiting GDF-15 significantly improves appetite and body weight (Breen DM et al., Cell Metabolism. 2020; 32:938-950).

Muscle loss is one of the biggest characteristics of cachexia, and is known to be caused by increased protein catabolism and decreased protein production due to overproduction of various cytokines. Symptoms of cachexia include muscle loss (sarcopenia) and significantly overlap with each other. Patients with cachexia mostly have muscle loss (sarcopenia), but not all patients showing muscle loss show cachexia symptoms. Clinically speaking, muscle loss (sarcopenia) can be considered a sign of cachexia. Among the causes of weight loss in the patients after surgery, sarcopenia and cachexia commonly occur mainly in elderly patients, and both phenomena are characterized by a decrease in muscle mass and function (Seungwan Ryu, J. Clin. Nutr. 2017; 9:2-6).

Currently, a progesterone formulation (megestrol acetate; megace) and steroids (dexamethasone, and prednisone), etc. are used for the treatment of cachexia. However, the steroids have no long-lasting therapeutic effect, and show side effects such as water retention, insulin resistance, and adrenal gland hypofunction, when used for a long period of time, and thus only short-term prescriptions of the steroids are possible. The progesterone formulation (megestrol acetate) was approved by the US FDA for use in AIDS patients showing loss of appetite or weight loss, and is currently most commonly prescribed for cancer cachexia patients, and thus it was used as a comparative drug in the present invention. However, the progesterone formulation increases adipose tissue rather than muscle, and has side effects such as thromboembolism, edema, erectile dysfunction, uterine bleeding, hyperglycemia, and adrenal gland hypofunction.

Patients with cachexia show a low response to anticancer therapy and experience serious side effects. Anticancer treatment methods for cancer patients broadly include surgical operation, chemotherapy, and radiotherapy. Surgical operation is a treatment method for removing a lesion (i.e., cancer). Early-stage cancer can be completely cured by surgical operation, but middle or later stage cancer cannot be cured by surgical operation only. Chemotherapy, radiotherapy, or combination therapy thereof is commonly used to remove cancer cells by cytotoxic action, but these therapies have low specificity, and thus also damage normal cells having characteristics of dividing and proliferating rapidly, such as hematopoietic cells or immune cells. Therefore, these therapies cause side effects such as vomiting symptoms, loss of appetite, stomatitis, diarrhea, constipation, fever, infection, leukopenia, thrombocytopenia, anemia, abdominal pain, nephrotoxicity, hapatotoxicity, cardiotoxicity, peripheral neurotoxicity, central nervous system toxicity, muscle pain, bone pain, bone marrow suppression, and the like. Cachexia and muscle loss, which occur at a high frequency in cancer patients, lower the rate of response to anticancer therapy, make it difficult to achieve effective anticancer therapy, and lower the patient's quality of life.

Paeoniflorin has been demonstrated to have antiinflammatory and antioxidant effects (Tao Y et al., Experimental and Therapeutic Medicine, 2016; 11: 263-268), an effect of preventing and treating infertility (Korean Patent Application Publication No. 10-2016-0121023), a memory enhancement effect (Korean Patent Application Publication No. 10-2016-0089930), and a fat reduction effect (Korean Patent Application Publication 10-2011-0138322), but the effects of paeoniflorin on the prevention, alleviation and treatment of cachexia and muscle loss have not yet been reported.

Therefore, in order to treat cachexia and muscle loss occurring in cancer and other diseases, there is a need to develop materials that are superior to existing therapeutic agents and safe for the human body.

### DISCLOSURE

### Technical Problem

One aspect provides a pharmaceutical composition for preventing or treating cachexia and muscle loss containing paeoniflorin as an active ingredient.

Another aspect provides a food composition for preventing or alleviating cachexia and muscle loss containing paeoniflorin.

Still another aspect provides an anticancer adjuvant for preventing or treating cachexia and muscle loss containing paeoniflorin.

### Technical Solution

One aspect provides a pharmaceutical composition for preventing or treating cachexia and muscle loss containing paeoniflorin as an active ingredient.

According to one embodiment, the cachexia may be caused by cancer.

According to one embodiment, the cachexia may be caused by an anticancer drug.

According to one embodiment, the composition may alleviate a symptom selected from the group consisting of weight loss, reduced muscle mass, reduced muscle strength, reduced endurance, reduced motor coordination, and increased pro-inflammatory cytokines and GDF-15.

According to one embodiment, the prevention or treatment of the cachexia and muscle loss may be achieved by inhibiting an increase in any one selected from the group consisting of TNF-α, IL-6, IL-1β, and GDF-15.

According to one embodiment, the paeoniflorin may be derived from any one plant species selected from the group consisting of *Paeonia suffruticosa* Andrews, *Paeonia lactiflora* Pall., *Paeonia lactiflora var. trichocarpa (Bunge)* Stern, *Paeonia lactiflora f. nuda* Nakai, *Paeonia lactiflora f. pilosella* Nakai, *Paeonia japonica (Makino)* Miyabe & Takeda, *Paeonia japonica var. pillosa* Nakai, *Paeonia obovata* Maxim, *Paeonia veitchii* Lynch, and other related plant species (Paeoniaceae).

Another aspect provides a food composition for preventing or alleviating cachexia and muscle loss containing paeoniflorin.

Still another aspect provides an anticancer adjuvant for preventing or treating cachexia and muscle loss containing paeoniflorin.

### Advantageous Effects

The pharmaceutical composition containing paeoniflorin according to one embodiment is able to alleviate weight loss, muscle mass loss, reduced muscle strength, reduced endurance, and reduced motor coordination, and inhibit an increase in pro=inflammatory cytokines and an increase in GDF-15, and thus may be useful for the prevention or treatment of cachexia and muscle loss.

### Brief Description of Drawings

FIG. 1 shows data obtained by measuring body weight (A), weight of gastrocnemius muscle (B), and weight of quadriceps muscle (C) after administering paeoniflorin to C57BL/6 mice with cisplatin-induced cachexia.
FIG. 2 shows data obtained by measuring body weight (A), weight of gastrocnemius muscle (B), and weight of quadriceps muscle (C) after administering paeoniflorin to Balb/c mice with cisplatin-induced cachexia.
FIG. 3 shows the results of testing skeletal muscle function after administration of paeoniflorin in C57BL/6 mice with cisplatin-induced cachexia. Specifically, it shows the results of measuring muscle strength (A), endurance (B), and motor coordination (C).
FIG. 4 shows the results of testing skeletal muscle function after administration of paeoniflorin in Balb/c mice with cisplatin-induced cachexia. Specifically, it shows the results of measuring muscle strength (A), and endurance (B).
FIG. 5 shows data obtained by measuring MuRF1 and MAFbx mRNA levels in the gastrocnemius muscle by real-time PCR after administration of paeoniflorin in C57BL/6 mice with cisplatin-induced cachexia.
FIG. 6 shows data obtained by measuring MuRF1 protein level (A), MAFbx protein level (B), Myosin heavy chain(MyHC) protein level (C), Myoblast determination protein 1(MyoD) protein level (D), and β-actin protein level (E) in the gastrocnemius muscle by Western blotting after administration of paeoniflorin in C57BL/6 mice with cisplatin-induced cachexia.
FIG. 7 shows the results of quantifying the intensities of protein bands, observed in Western blotting of MyHC (A) and MyoD (B) in the gastrocnemius muscle, using ImageJ software (NIH, Bethesda, MD, USA) after administration of paeoniflorin in C57BL/6 mice with cisplatin-induced cachexia.
FIG. 8 shows data obtained by measuring and comparing the mRNA expression levels of TNF-α (A), IL-6 (B), IL-1β (C), IL-1α (D), CCL2 (E), and CCL5 (F) in the gastrocnemius muscle after administration of paeoniflorin in C57B/6 mice with cisplatin-induced cachexia.
FIG. 9 shows data obtained by measuring and comparing protein levels of TNF-α (A), IL-6 (B), and IL-1β (C) in the gastrocnemius muscle after administration of paeoniflorin in Balb/c mice with cisplatin-induced cachexia.
FIG. 10 shows the results of quantifying the levels of p-IKK (phospho-IKK) (A) and p-IκB (phospho-IκB) (B) in the gastrocnemius muscle by ImageJ software analysis of Western blotting protein bands after administration of paeoniflorin in C57B/6 mice with cisplatin-induced cachexia.
FIG. 11 shows the results of quantifying the level of IκB in the gastrocnemius muscle by ImageJ software analysis of Western blotting protein band after administration of paeoniflorin in C57B/6 mice with cisplatin-induced cachexia.
FIG. 12 shows the results of quantifying the cytosolic NF-κB level (A) and the nuclear NF-κB level (B) in the gastrocnemius muscle by ImageJ software analysis of Western blotting protein bands after administration of paeoniflorin in C57B/6 mice with cisplatin-induced cachexia. It also shows data obtained by quantifying the target DNA binding activity of NF-κB (C) in in the gastrocnemius muscle.
FIG. 13 shows the results of measuring the plasma concentrations of TNF-α (A) and GDF-15 (B) by ELISA after administering paeoniflorin to C57B/6 mice with cisplatin-induced cachexia.
FIG. 14 shows the results of measuring body weight (A), weight of gastrocnemius muscle (B), and weight of quadriceps muscle (C) after administering paeoniflorin to C57BL/6 mice with Lewis lung carcinoma (LLC)-induced cachexia.
FIG. 15 shows the results of measuring body weight (A), weight of gastrocnemius muscle (B), and weight of quadriceps muscle (C) after administering paeoniflorin to Balb/c mice with colon-26 carcinoma(C26)-induced cachexia.
FIG. 16 shows the results of measuring muscle strength (A) and endurance (B) after administering paeoniflorin to C57BL/6 mice with Lewis lung carcinoma-induced cachexia.
FIG. 17 shows the results of measuring muscle strength (A) and endurance (B) after administering paeoniflorin to Balb/c mice with colon-26 carcinoma-induced cachexia.
FIG. 18 shows the results of measuring the plasma concentration of GDF-15 by ELISA after administering paeoniflorin to C57BL/6 mice with Lewis lung carcinoma-induced cachexia.
FIG. 19 depicts photographs showing the results of observing the effect of paeoniflorin on promotion of myotube differentiation of C2C12 myoblasts.
FIG. 20 shows the results of quantitatively analyzing the effect of paeoniflorin on promotion of myotube differentiation of C2C12 myoblast by the ImageJ program.

### Best Mode

To achieve the above object, one aspect of the present invention provides a pharmaceutical composition for preventing or treating cachexia and muscle loss containing paeoniflorin.

As used herein, the term "cachexia" refers to a high degree of general weakness that can be seen at the end stage of cancer, tuberculosis, diabetes, acquired immunodeficiency syndrome (AIDS), and the like. Cachexia is frequently seen in patients with gastrointestinal cancer, such as gastric cancer, esophageal cancer or colorectal cancer, and lung cancer patients. Cachexia shows symptoms such as loss of appetite, loss of weight and stamina, muscle loss, anemia, lethargy, and indigestion. In particular, cachexia shows either a condition in which normal food ingestion is difficult due to loss of appetite, or a condition in which body weight and muscle are lost even though normal food ingestion is possible. When cachexia occurs, it shows low response to anticancer chemotherapy or radiotherapy, and thus reduces the patient's quality of life, results in shortening of life expectancy, and causes death due to cachexia in 20 to 30% of all cancer patients.

As used herein, the term "muscle loss" refers to a condition in which muscles in the body are decreased. For example, the muscle loss may be a decrease in muscle tissue caused by not using muscle, a decrease in muscle tissue caused by a disease of the muscle itself, or a decrease in muscle tissue caused by damage to a nerve that controls the muscle. This muscle loss may be caused by, for example, cachexia.

According to one embodiment of the present invention, the "paeoniflorin" may be a compound represented by the following Formula 1:

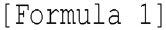

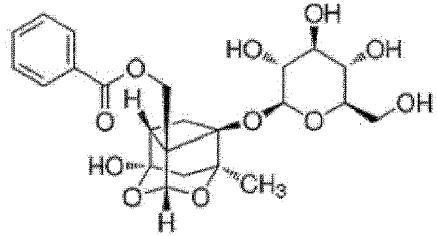

According to one embodiment of the present invention, the cachexia may be caused by cancer. Meanwhile, the cachexia may be caused by an anticancer drug used for cancer treatment. The anticancer drug may be selected from the group consisting of cisplatin, doxorubicin, irinotecan, paclitaxel, daunorubicin, docetaxel, and 5-fluorouracil, without being limited thereto.

Paeoniflorin used as an active ingredient of the composition of the present invention is extracted from Paeoniae radix. Paeoniae radix satisfies the standards of the Korean Pharmacopoeia, and the root of *Paeonia lactiflora* Pall., which is a plant belonging to the family Paeoniaceae, is used as a medicinal herb. It is known that Paeoniae radix has an antibacterial effect, inhibits the growth of *Staphylococcus aureus* and *Shigella* bacteria, and is effective against abdominal pain and diarrhea. However, the effects of Paeoniae radix on the inhibition of muscle loss and the alleviation of cachexia have not been known.

According to one embodiment, the paeoniflorin may be isolated from a water or ethanol extract of *Paeonia lactiflora* Pall.

The paeoniflorin may be isolated from an extract of any one plant species selected from the group consisting of *Paeonia suffruticosa* Andrews, *Paeonia lactiflora* Pall., *Paeonia lactiflora var. trichocarpa (Bunge)* Stern, *Paeonia lactiflora f. nuda* Nakai, *Paeonia lactiflora f. pilosella* Nakai, *Paeonia japonica (Makino)* Miyabe & Takeda, *Paeonia japonica var. pillosa* Nakai, *Paeonia obovata* Maxim, *Paeonia veitchii* Lynch, and other related plant species (Paeoniaceae). Preferably, it may be isolated from an extract of *Paeonia lactiflora* Pall.

According to one embodiment of the present invention, the extract of *Paeonia lactiflora* Pall. may be prepared according to a conventional method known in the art. For example, the Paeoniae radix extract may be prepared by pulverizing Paeoniae radix, adding thereto a solvent which is commonly used for extraction, and performing extraction at an appropriate temperature and pressure.

According to one embodiment of the present invention, the solvent may be selected from the group consisting of distilled water, a C₁ to C₄ lower alcohol, hexane, ethyl acetate, chloroform, diethyl ether, dichloromethane, acetone, and mixtures thereof.

Meanwhile, the Paeoniae radix extract includes not only the extract obtained by the solvent extraction method described above, but also an extract obtained through a conventional purification process. For example, the Paeoniae radix extract of the present invention may also include fractions obtained by additionally performing various purification processes, such as separation using an ultrafiltration membrane having a certain molecular weight cut-off value, or separation by various chromatography systems (manufactured for separation according to size, charge, hydrophobicity or affinity). In addition, the Paeoniae radix extract may be prepared in a powder form by additional processes such as reduced pressure distillation and freeze drying or spray drying.

According to one embodiment of the present invention, the paeoniflorin is able to alleviate a symptom selected from the group consisting of weight loss, reduced muscle mass, reduced muscle strength, inhibited myotube differentiation, reduced endurance, reduced motor coordination, and increased pro-inflammatory cytokines and GDF-15, and thus may be useful for the prevention, alleviation and treatment of cachexia and muscle loss. In addition, the paeoniflorin can significantly improve appetite and weight by suppressing the increase in GDF-15, a type of cytokine that has recently been shown to cause loss of appetite and weight loss due to tumors and chemotherapy, and can promote myotube differentiation, and thus it may be useful for the prevention, alleviation and treatment of cachexia and muscle loss.

For administration, the pharmaceutical composition may be preferably formulated as a pharmaceutical composition further containing one or more pharmaceutically acceptable carriers, in addition to the paeoniflorin described above.

The dosage form of the pharmaceutical composition may be granules, powders, tablets, coated tablets, capsules, suppositories, solutions, syrups, juices, suspensions, emulsions, drops, or injectable solutions. For example, for formulation in the form of tablets or capsules, the active ingredient may be combined with an oral, non-toxic pharmaceutically acceptable inert carrier such as ethanol, glycerol or water. In addition, if desired or necessary, suitable binders, lubricants, disintegrants and dyes may likewise be incorporated into the mixture. Suitable binders include, but are not limited to, starch, gelatin, natural sugars such as glucose or beta-lactose, maize sweeteners, natural and synthetic rubbers such as acacia, tragacanth gum or sodium oleate, sodium stearate, magnesium stearate, sodium benzoate, sodium acetate, sodium chloride, and the like. Disintegrants include, but are not limited to, starch, methyl cellulose, agar, bentonite, xanthan gum, and the like.

As an acceptable pharmaceutical carrier in a composition which is formulated as a liquid solution, it is possible to use one or more of saline, sterile water, Ringer's solution, buffered saline, albumin solution for injection, dextrose solution, maltodextrin solution, glycerol, ethanol, and mixtures thereof, which are suitable for sterilization and *in vivo* use, and other conventional additives such as antioxidants, buffers, bacteriostatic agents, etc. may be added as needed. In addition, the pharmaceutical composition may be formulated as injectable formulations such as an aqueous solution, suspension or emulsion, pills, capsules, granules or tablets by additionally adding a diluent, a dispersant, a surfactant, a binder, and a lubricant.

The pharmaceutical composition of the present invention may be administered orally or parenterally. For parenteral administration, the pharmaceutical composition may be administered by intravenous injection, subcutaneous injection, intramuscular injection, intraperitoneal injection, transdermal administration, or the like.

A suitable dose of the pharmaceutical composition of the present invention may be determined depending on factors such as formulation method, administration mode, the patient's age, weight, sex, pathological condition and diet, administration time, administration route, excretion rate, and response sensitivity.

According to one embodiment of the present invention, the daily dose of the pharmaceutical composition of the present invention may be 1 to 10,000 mg/kg, 1 to 1,000 mg/kg, or 1 to 100 mg/kg, preferably 10 or 20 mg/kg. The daily dose for humans may be determined by multiplying the above dose value by 0.081, which is the human equivalent dose factor (HEDF). That is, the daily dose for humans may be 0.081 to 810 mg/kg, 0.081 to 81 mg/kg, or 0.081 to 8.1 mg/kg, preferably 0.81 to 1.62 mg/kg.

The pharmaceutical composition of the present invention may be prepared in a unit dose form or prepared to be contained in a multi-dose container by formulating with a pharmaceutically acceptable carrier and/or excipient, according to a method that may be easily carried out by those skilled in the art.

Another aspect provides a food composition for preventing or alleviating cachexia and muscle loss containing paeoniflorin as an active ingredient.

The food composition according to the present invention may be used as a functional food obtained by formulation in the same manner as the pharmaceutical composition, or may be added to various foods. Foods to which the composition of the present invention may be added include, for example, beverages, confectionery, diet bars, dairy products, meats, chocolate, pizza, ramen, other noodles, gums, ice creams, vitamin complexes, and health food supplements.

The food composition of the present invention may contain ingredients that are commonly added in food production, in addition to containing paeoniflorin as an active ingredient. For example, the food composition contains proteins, carbohydrates, fats, nutrients, seasoning agents, and flavoring agents. Examples of the carbohydrates include conventional sugars, such as monosaccharides (e.g., glucose, fructose, etc.), disaccharides (e.g., maltose, sucrose, oligosaccharides, etc.), polysaccharides (e.g., dextrin, cyclodextrin, etc.), and sugar alcohols such as xylitol, sorbitol, erythritol or the like. Examples of the flavoring agents that may be used include natural flavoring agents (thaumatin, stevia extracts, such as rebaudioside A, glycyrrhizin, etc.), and synthetic flavoring agents (saccharin, aspartame, etc.). For example, when the food composition of the present invention is prepared as a drink or a beverage, it may further contain citric acid, liquid fructose, sugar, glucose, acetic acid, malic acid, fruit juice, and various plant extracts, in addition to paeoniflorin of the preset invention.

Still another aspect provides an anticancer adjuvant containing paeoniflorin as an active ingredient.

As used herein, the term "anticancer adjuvant" refers to an adjuvant exhibiting the effect of alleviating the side effects of an anticancer drug during anticancer treatment. The side effects of the anticancer drug include hematopoietic toxicity, loss of appetite, weight loss, and the like.

The anticancer adjuvant may further contain one or more active ingredients that exhibit the same or similar functions, in addition to containing the paeoniflorin as an active ingredient. The anticancer adjuvant may be administered orally or parenterally during clinical administration. For parenteral administration, the anticancer adjuvant may be administered by intraperitoneal injection, intrarectal injection, subcutaneous injection, intravenous injection, intramuscular injection, intrauterine, intradural injection, intracerebrovascular injection, or intrathoracic injection, and may be used in the form of a general pharmaceutical formulation.

The anticancer adjuvant may be used alone or in combination with surgery, radiotherapy, hormone therapy, chemotherapy, and methods using a biological response modifier.

The daily dose of the anticancer adjuvant may be about 0.0001 to 1,000 mg/kg, 0.001 to 100 mg/kg, 0.01 to 100 mg/kg, 0.1 to 100 mg/kg, or 1 to 100 mg/kg, preferably 10 or 20 mg/kg.

The daily dose for humans may be determined by multiplying the above dose value by 0.081, which is the human equivalent dose factor (HEDF). That is, the daily dose for humans may be 8.1×10⁻⁶ to 81 mg/kg, 8.1×10⁻⁵ to 8.1 mg/kg, or 8.1×10⁻⁴ to 8.1 mg/kg, preferably 0.81 to 1.62 mg/kg.

Although the anticancer adjuvant is preferably administered once or several times a day, the range of the daily dose of the anticancer adjuvant varies depending on the patients' body weight, age, sex, health condition and diet, administration time, administration mode, excretion rate, and disease severity. The anticancer adjuvant of the present invention may be administered in various parenteral formulations at the time of actual clinical administration. For formulation, the anticancer adjuvant is formulated using diluents or excipients which are commonly used, such as fillers, extenders, binders, wetting agents, disintegrants, surfactants, etc. Formulations for parenteral administration include sterilized aqueous solutions, non-aqueous solutions, suspensions, emulsions, freeze-dried preparations, and suppositories. As non-aqueous solvents or suspending agents, it is possible to use propylene glycol, polyethylene glycol, vegetable oils such as olive oil, injectable esters such as ethyl oleate, and the like. As a base for suppositories, it is possible to use witepsol, Macrogol, Tween 61, cacao butter, laurin fat, glycerogelatin, and the like.

Yet another aspect of the present invention provides a method of preventing or treating cachexia and muscle loss by administering a pharmaceutical composition containing paeoniflorin.

The pharmaceutical composition containing paeoniflorin according to the present invention can alleviate weight loss, muscle loss, and fatigue, and thus may be useful for the prevention or treatment of cachexia and muscle loss.

The composition containing paeoniflorin according to the present invention can inhibit the increase in any one selected from the group consisting of TNF-α, IL-6, IL-1β, and GDF-15, and thus may be useful for the prevention and treatment of cachexia and muscle loss.

### Mode for Invention

Hereinafter, one or more embodiments will be described in more detail by way of examples. However, these examples are intended to illustrate one or more embodiments, and the scope of the present invention is not limited to these examples.

### Example 1. Material Preparation - Preparation of Paeoniflorin

### 1-1. Preparation of Paeoniae Radix Extract

The roots of *Paeonia lactiflora* Pall. were pulverized. The pulverized material was extracted with a 5-fold volume of an extraction solvent consisting of 75% ethanol and water three times for 3 hours each time, and the extract was concentrated. After removing the remaining ethanol from the concentrate, the residue was dissolved by heating in an equal volume of water. The solution was fractionated three times with an equal volume of ethyl ether, and the aqueous layer was collected and then concentrated. The concentrate was loaded onto a 200-300 mesh silica gel column, separated using chloroform: acetone (4:1) as a mobile phase, concentrated, and dried.

### 1-2. Isolation of Paeoniflorin

The dried material was suspended again in water, and the suspension was loaded onto a column (10 mm diameter × 250 mm length) packed with 4 µm octadecyl silylated silica gel. The mobile phase used was a mixed solvent of 0.5% TFA (trifluoroacetic acid) and methanol at a volume ratio of 75:25. A UV detector (230 nm) was used for detection of paeoniflorin, the mobile phase was flowed at a rate of 2.5 ml/min, the major peak detected at 18 to 20 minutes was repeatedly separated, and the obtained fraction was distilled under reduced pressure to obtain paeoniflorin. The obtained paeoniflorin was analyzed for mass by an LCQ mass spectrometer (Finnigan, USA) and qualitatively analyzed by NMR (Bruker).

Table 1 below shows the mass spectrometry results, and Table 2 below shows the NMR results. Through these results, paeoniflorin (molecular formula: C₂₃H₂₈O₁₁) of Formula 1 could be identified.

**[Table 1]**

| Sample | [M+Na]+ | Estimated molecular weight | Remarks |
|---|---|---|---|
| Paeoniflorin | 503 | 480 | 983=[2M+Na]+ |

**[Table 2]**

| | Classification | ¹H | ¹³C |
|---|---|---|---|
| 1 | Q | - | 89.3 |
| 2 | CH₂ | 2.48, 1.95 | 23.3 |
| 3 | CH | 2.59 | 43.9 |
| 4 | Q | - | 106.3 |
| 5 | CH₂ | 2.19, 1.82 | 44.4 |
| 6 | Q | - | 87.2 |
| 7 | Q | - | 71.6 |
| 8 | CH₃ | 1.36 | 19.6 |
| 10 | CH₂ | 1.64, 1.53 | 102.2 |
| 12 | CH₂ | 1.51, 1.40 | 62.8 |
| 1' | Q | - | 131.1 |
| 2' | CH * 2 | 8.04 | 130.6 |
| 3' | CH * 2 | 7.47 | 129.6 |
| 4' | CH | 7.60 | 134.4 |
| 1" | CH | 4.53 | 100.1 |
| 2" | CH | 3.37-3.18 | 77.8 |
| 3" | CH | 3.37-3.18 | 77.9 |
| 4" | CH | 3.37-3.18 | 72.1 |
| 5" | CH | 3.37-3.18 | 74.9 |
| 6" | CH | 3.85, 3.61 | 61.7 |

The structural formula of paeoniflorin is shown in Formula 1 below.

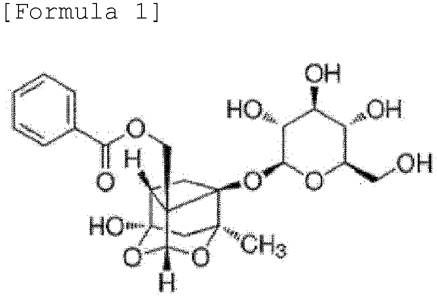

### Example 2. Evaluation of Effect on Alleviation of Cachexia and Muscle Loss Induced by Anticancer Drug and Investigation of Mechanism of Action Thereof

### 2-1. Experimental Methods

### 2-1-1. Preparation of Reagents

Anti-IκB (cat# 9242), anti-phospho-IκB (Ser 32/36) (cat# 9246), anti-phospho-IKK (cat# 2694S), IKK (cat# 7218) and anti-NF-κB p65 (cat# 8242) primary antibodies along with anti-mouse (cat# 7076S) and anti-rabbit (cat# 7074S) secondary antibodies were purchased from Cell Signaling Technology (Danvers, MA, USA). Anti-MuRF1 (cat# 172479) and anti-MAFbx (cat# 157596) antibodies were obtained from Abeam (Cambridge, UK). Anti-myosin heavy chain (MyHC) antibody (cat# MAB4470) was purchased from R&D Systems (Minneapolis, MN, USA).

Anti-myoblast determination protein (MyoD) (cat# 377460), anti-β-actin (cat# 47778), and anti-TATA box binding protein (TBP, cat# 204) antibodies were from Santa Cruz Biotechnology, Inc. (Santa Cruz, CA, USA). Megestrol acetate (MA; Megace) was purchased from Tokyo chemical industry (Tokyo, Japan).

### 2-1-2. Preparation of Animal Experiments

The animal experiments were performed in accordance with internationally accepted principles for laboratory animal use and care, and the corresponding study protocol was reviewed and approved by the Institutional Animal Care and Use Committee (IACUC) at Chung-Ang University (approval# 2021-00022). Four-week-old female C57BL/6 mice and Balb/c mice were purchased from Raonbio Inc. (Yongin, Korea) and were provided with standard laboratory diet and water *ad libitum* in the animal facility of Chung-Ang University. Each experimental group for each mouse type consisted of 10 mice treated as shown in Tables 3 and 4 below. After a week of acclimation, the mice were intraperitoneally injected three times with 3 mg/kg/day cisplatin, suspended in 200 µL saline, every other day. Control group mice (sham-treated mice) were injected with the same volume of saline under the same regimen. The cisplatin-treated groups were orally administered paeoniflroin (Pae) or megestrol acetate (MA), suspended in 200 µL distilled water, every day until euthanasia.

Specifically, the cisplatin-treated groups were orally administered paeoniflorin (prepared in Example 1) at a dose of 5 (Pae 5 group), 10 (Pae 10 group), or 20 (Pae 20 group) mg/kg once daily for 9 days. The comparative drug-treated group was orally administered megestrol acetate (MA), which is currently most commonly prescribed for cancer cachexia patients in clinical practice, at a dose of 160 mg/kg (= 800 mg [daily human dose]/60 kg [average human body weight] × 12 [dose equivalent factor based on human: mouse body surface area ratio], once daily for 9 days.

In addition, cisplatin-treated mice orally administered the same volume of distilled water were designated as the vehicle group. After completion of drug treatment, the mice of each group were euthanized with 10 mg/kg alfaxalone (Jurox, Rutherford, Australia). Blood was collected in test tubes containing EDTA, and centrifuged, and plasma was separated therefrom. Gastrocnemius and quadriceps skeletal muscles were dissected and weighed. The skeletal muscles were rapidly frozen in liquid nitrogen to be used for RT-qPCR, Western blotting, and ELISA.

**[Table 3]**

| Group (C57BL/6 mice) | Treatment |
|---|---|
| Control group (control) | Intraperitoneally administered 200 µL of saline three times every other day and orally administered 200 µL of distilled water daily |
| Negative control group (vehicle) | Intraperitoneally administered cisplatin, suspended in 200 µL of saline, at a dose of 3 mg/kg/day three times every other day and orally administered 200 µL of distilled water daily |
| Paeoniflorin 10 (Pae 10) | Intraperitoneally administered cisplatin, suspended in 200 µL of saline, at a dose of 3 mg/kg/day three times every other day, and orally administered paeoniflorin at a dose of 10 mg/kg daily |
| Paeoniflorin 20 (Pae 20) | Intraperitoneally administered cisplatin, suspended in 200 µL of saline, at a dose of 3 mg/kg/day three times every other day, and orally administered paeoniflorin at a dose of 20 mg/kg daily |
| Positive control group (MA) | Intraperitoneally administered cisplatin, suspended in 200 µL of saline, at a dose of 3 mg/kg/day three times every other day, and orally administered MA (megestrol acetate) at a dose of 160 mg/kg daily |

**[Table 4]**

| Group (Balb/c mice) | Treatment |
|---|---|
| Control group (control) | Intraperitoneally administered 200 µL of saline three times every other day and orally administered 200 µL of distilled water daily |
| Negative control group (vehicle) | Intraperitoneally administered cisplatin, suspended in 200 µL of saline, at a dose of 3 mg/kg/day three times every other day and orally administered 200 µL of distilled water daily |
| Paeoniflorin 5 (Pae 5) | Intraperitoneally administered cisplatin, suspended in 200 µL of saline, at a dose of 3 mg/kg/day three times every other day, and orally administered paeoniflorin at a dose of 5 mg/kg daily |
| Paeoniflorin 10 (Pae 10) | Intraperitoneally administered cisplatin, suspended in 200 µL of saline, at a dose of 3 mg/kg/day three times every other day, and orally administered paeoniflorin at a dose of 10 mg/kg daily |
| Paeoniflorin 20 (Pae 20) | Intraperitoneally administered cisplatin, suspended in 200 µL of saline, at a dose of 3 mg/kg/day three times every other day, and orally administered paeoniflorin at a dose of 20 mg/kg daily |
| Positive control group (MA) | Intraperitoneally administered cisplatin, suspended in 200 µL of saline, at a dose of 3 mg/kg/day three times every other day, and orally administered MA (megestrol acetate) at a dose of 160 mg/kg daily |

### 2-1-3. Skeletal Muscle Function Test

A skeletal muscle function test was conducted one day before euthanasia. Muscle strength of each mouse was measured by the grip strength of front paws, and endurance of each mouse was measured by treadmill running time until exhaustion. Motor coordination was measured by retention time on a rotating cylinder with a diameter of 3 cm (Castro and Kuang, 2017) . The grip strength of each mouse was measured using a grip strength meter (Bio-GS3, Bioseb, Vitrolles, France). The treadmill running time of each mouse was measured on a treadmill (Exer-3/6, Columbus Instruments, Baltimore, MD, USA). The ability of each mouse to stay on a rotating cylinder was measured using a rotarod apparatus (Panlab, Barcelona, Spain).

### 2-1-4. Measurement of mRNA Levels in Skeletal Muscle

Total RNA was isolated from skeletal muscle using Qiazol (Qiagen, Germany), and then the mRNA levels of pro-inflammatory cytokines and muscle-specific ubiquitin E3 ligases (MuRF1 and MAFbx) were measured by real-time PCR.

### 2-1-5. Measurement of Protein Levels

The levels of TNF-α and interleukin (IL)-6 were measured using respective mouse ELISA kits (Invitrogen, Waltham, MA, USA). IL-1β and GDF-15 levels were measured using respective Quantikine ELISA kits (R&D Systems, Minneapolis, MN, USA).

### 2-1-6. Western Blotting

Proteins were extracted from skeletal muscle using NucleoSpin kits (Macherey-Nagel, Duren, Germany) and quantified using the BCA Protein Assay Kit (Pierce, Rockford, IL, USA). Anti-β-actin antibody was used to quantify protein loading. For myosin protein (MyHC, myosin heavy chain), myogenic factor (MyoD), and NF-κB signaling system (p-IKK, IKK, p-IκB, and IkB), protein levels in the gastrocnemius muscle were measured by Western blotting. Band intensities were quantified using ImageJ software (NIH, Bethesda, MD, USA) .

### 2-1-7. Measurement of Nuclear and Cytoplasmic NF-κB Levels and Target DNA Binding Activity

Nuclear and cytoplasmic lysates were prepared from the skeletal muscle using a nuclear extraction kit (Active Motif, Carlsbad, CA, USA). The protein concentration of both lysates was measured using the BCA Protein Assay Kit (Pierce). Anti-NF-κB p65 was used as the primary antibody, and 10 µg of protein per lysate was analyzed by Western blotting. TBP (TATA box-binding protein) and β-actin were as controls for the nuclear and cytosolic lysates, respectively. The target DNA binding activity of NF-κB was measured using the TransAM NF-κB ELISA kit (Active Motif, USA).

### 2-1-8. Statistical Analysis

All data were obtained through at least three replicates and expressed as mean ± standard deviations. Statistically significant differences between experimental groups were assessed using unpaired t-tests. A P-value <0.05 was considered statistically significant. All analyses were performed using SPSS version 18.0 software (SPSS Inc., Chicago, IL, USA).

### 2-2. Evaluation of Effect of Paeoniflorin on Restoration of Body Weight and Muscle Mass in Cisplatin-Treated Mice

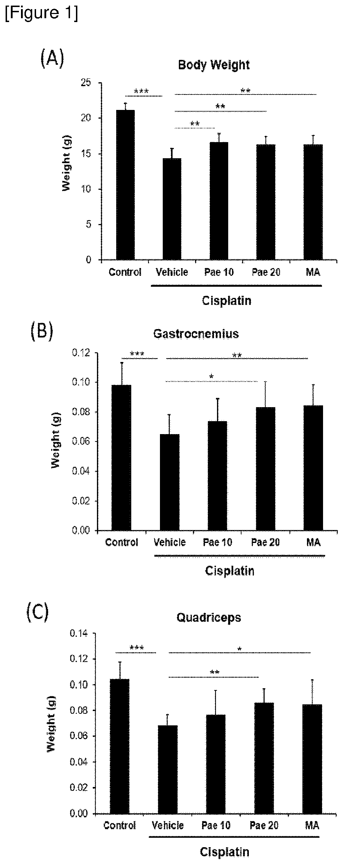

**As** shown in FIG. 1A, in the C57BL/6 mouse experiment, the body weight of the negative control group (vehicle) was significantly lower than that of the control group (control). However, when comparing the results of the Pae 10 and Pae 20 groups, which were the paeoniflorin-administered groups, it was confirmed that cisplatin-induced weight loss was attenuated by treatment with paeoniflorin.

As shown in FIGS. 1B and 1C, skeletal muscle masses, measured as the weight of the gastrocnemius and quadriceps muscles, were significantly lower in the cisplatin-administered mouse group than in the control mouse group. However, it was found that, when paeoniflorin was orally administered to the cisplatin-treated mice, the skeletal muscle mass was restored. *P<0.05, **P<0.01, ***P<0.001 (same as below)

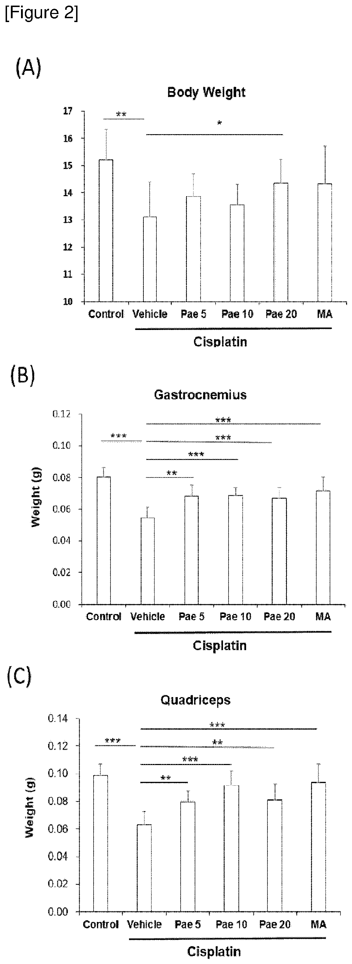

It was found that similar results were obtained in the experiment conducted using Balb/c mice. As shown in FIGS. 2A, 2B, and 2C, it was found that the body weight of the mice and the weights of the gastrocnemius and quadriceps muscles increased in the paeoniflorin-treated groups, Pae 5, Pae 10, and Pae 20, compared to those in the negative control group.

### 2-3. Evaluation of Effect of Paeoniflorin on Restoration of Skeletal Muscle Function in Cisplatin-Treated Mice

In C57BL/6 mice, skeletal muscle functions were measured by grip strength (muscle strength), treadmill running time (endurance), and rotarod retention time (motor coordination).

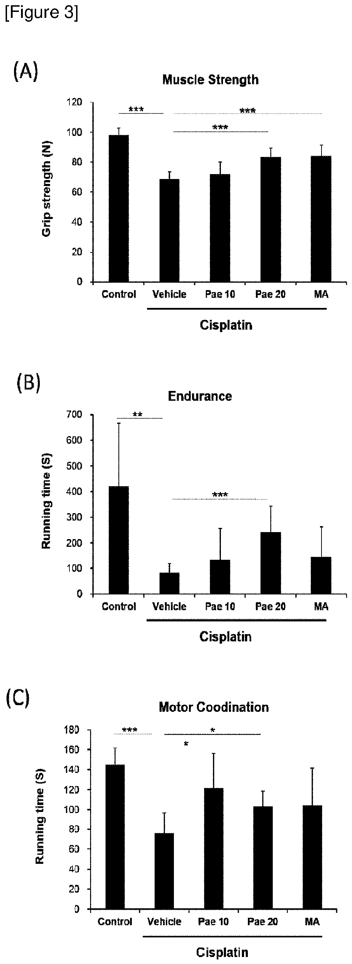

As shown in FIG. 3, skeletal muscle functions were significantly reduced by cisplatin treatment. However, skeletal muscle functions were significantly restored by the oral administration of paeoniflorin in the cisplatin-treated mice.

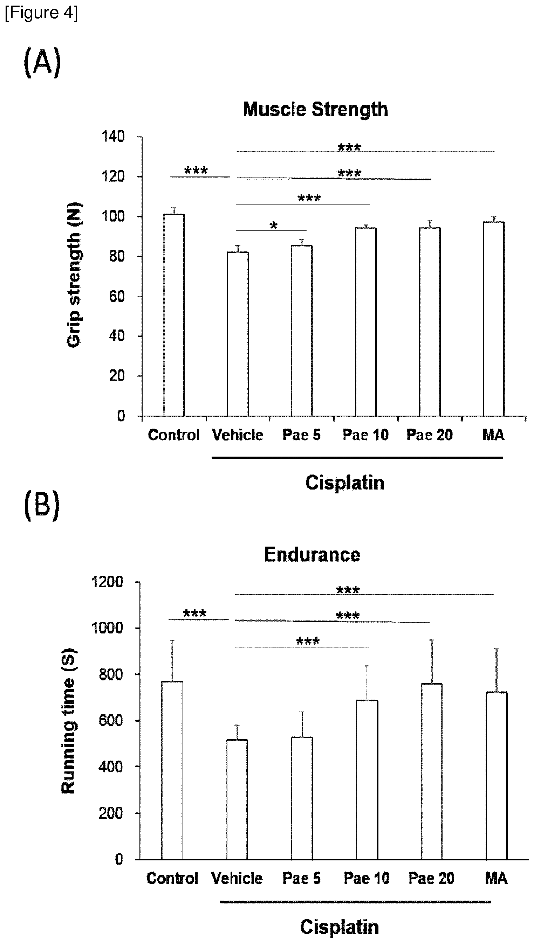

In addition, as shown in FIG. 4, it was found that paeoniflorin similarly restored muscle strength and endurance in Balb/c mice.

### 2-4. Evaluation of Effect of Paeoniflorin on Suppression of Muscle-Specific Ubiquitin E3 Ligases and Restoration of Proteins Involved in Skeletal Muscle Contraction and Differentiation

In order to measure the mRNA and protein levels of major factors involved in muscle loss, muscle contraction, and muscle differentiation, the mRNA and protein levels in the skeletal muscle tissues from C57BL/6 mice were measured by real-time PCR and Western blotting.

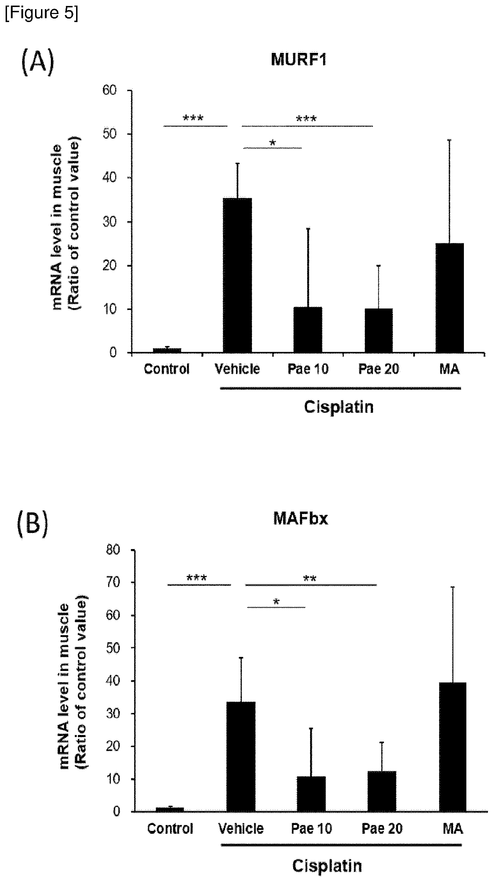

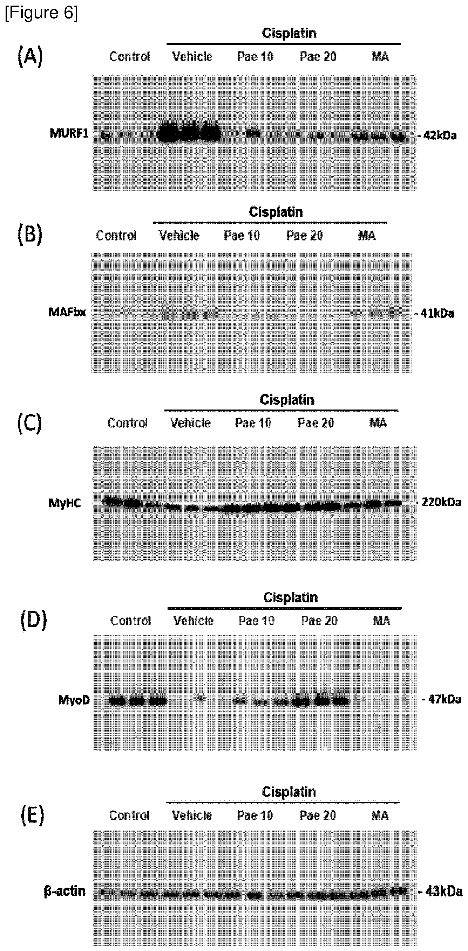

As shown in FIG. 5, it was found that the mRNA levels of muscle-specific ubiquitin E3 ligases (MuRF1 and MAFbx) increased in the negative control group (vehicle), but decreased in the paeoniflorin-treated groups (Pae 10 and Pae 20). As shown in FIGS. 6A and 6B, it was found that the protein levels of MuRF1 and MAFbx also increased in the negative control group (vehicle), but decreased in the paeoniflorin-treated groups (Pae 10 and Pae 20).

It is known that MuRF1 and MAFbx are involved in the ubiquitination and proteolysis of myosin heavy chain (MyHC), which plays a major role in muscle contraction, as well as myoblast determination protein(MyoD), which is a major transcription factor involved in muscle differentiation (Bodine & Baehr, Am J Physiol Endocrinol Metab, 307: E469-E484, 2014).

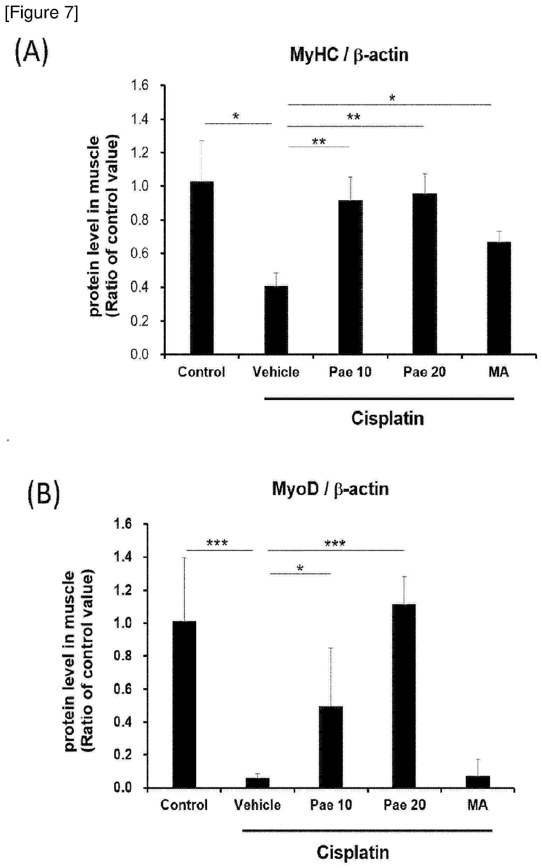

As shown in the Western blots of FIGS. 6C and 6D and in FIG. 7, it was found that the protein levels of MyHC and MyoD were reduced in the skeletal muscle of the negative control group (vehicle) compared to that of the control group (control). On the other hand, it was found that the protein levels of MyHC and MyoD increased in the paeoniflorin-treated groups (Pae 10 and Pae 20) compared to the negative control group (vehicle).

The increased intramuscular level of MyHC may be directly responsible for the paeoniflorin-induced increase in muscle function, such as grip strength and treadmill running time, and the increased intramuscular level of MyoD may be directly responsible for the paeoniflorin-induced increase in muscle mass.

The above results suggest that the decreases in MuRF1 and MAFbx and the increases in MyoD and MyHC are involved in the mechanism by which paeoniflorin exhibits the effect of improving muscle mass and muscle function.

### 2-5. Evaluation of Effect of Paeoniflorin on Reduction of Pro-inflammatory Cytokine and Chemokine Levels in Skeletal Muscle

The mRNA levels of pro-inflammatory cytokines such as TNF-α, IL-6, IL-1β, and IL-1α, as well as chemokines such as CCL2 and CCL5, in the skeletal muscle, were measured by real-time PCR.

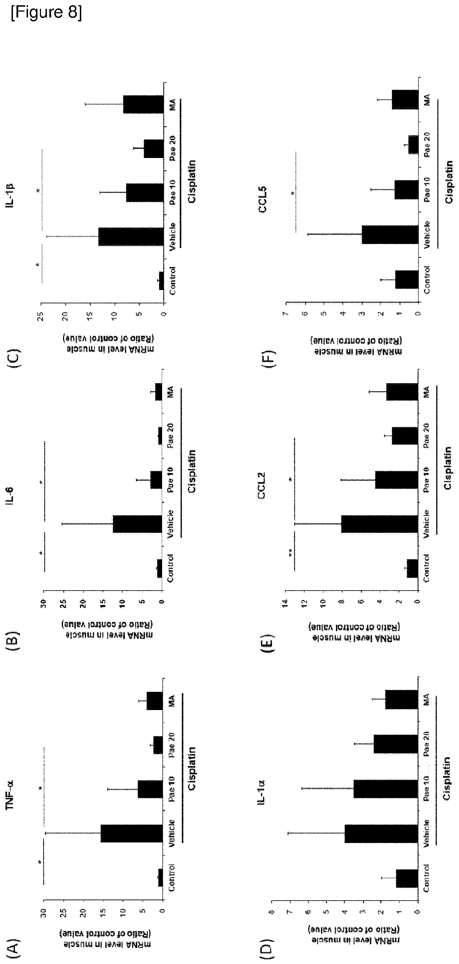

As shown in FIG. 8, in the results of the experiment performed using C57BL/6 mice, it was found that the expression levels of pro-inflammatory cytokines and chemokines in the muscle increased in the negative control group (vehicle), but the expression levels of pro-inflammatory cytokines and chemokines in the muscle decreased in the paeoniflorin-treated groups (Pae 10 and Pae 20) compared to the negative control group(vehicle).

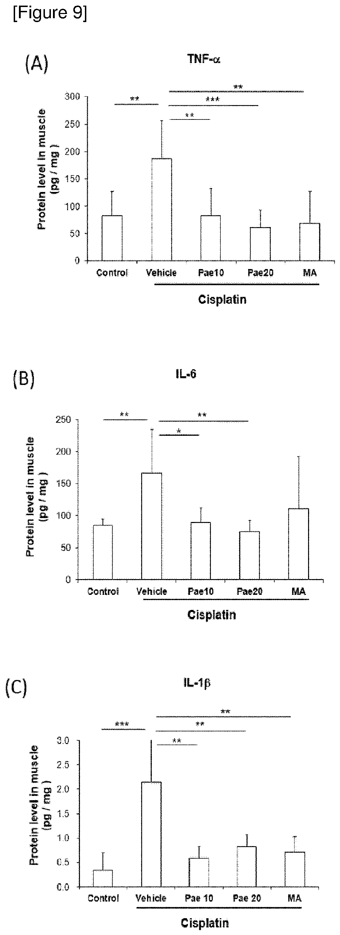

In addition, as shown in FIG. 9, the results of the experiment conducted on Balb/c mice confirmed that the effect against the expression of pro-inflammatory cytokine was similarly obtained.

### 2-6. Evaluation of Effect of Paeoniflorin on Inhibition of NF-κB Signaling in Skeletal Muscle

NF-κB signaling was analyzed by Western blotting of the total extract and the cytosolic and nuclear fraction from the skeletal muscle of C57BL/6 mice.

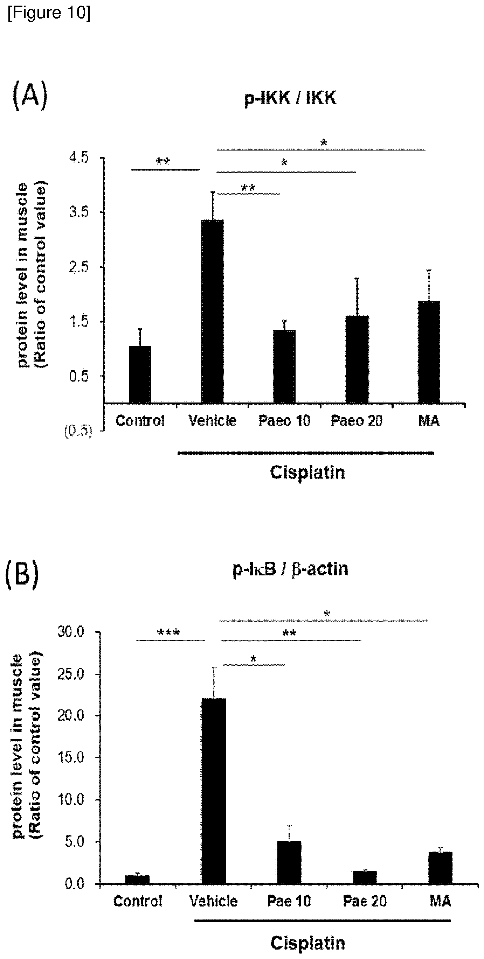

As shown in FIG. 10, it was found that the levels of p-IKK (phospho-IKK) and p-IκB (phospho-IκB) significantly increased in the negative control group (vehicle) compared to the control group (control), but the levels of p-IKK (phospho-IKK) and p-IκB (phospho-IκB) were reduced again in the paeoniflorin-treated groups (Pae 10 and Pae 20).

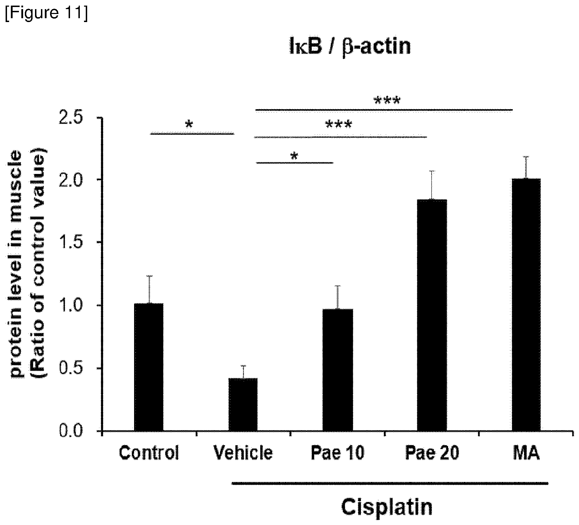

As shown in FIG. 11, it was found that the level of IκB was reduced in the negative control group (vehicle) compared to the control group (control), and the level of IκB increased in the paeoniflorin-treated groups (Pae 10 and Pae 20).

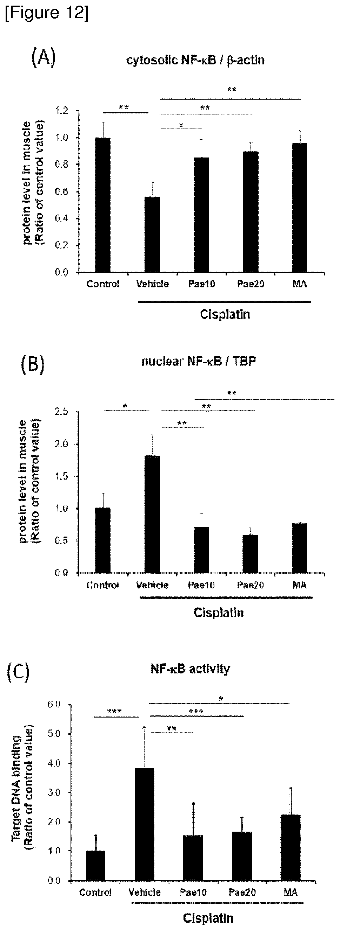

In addition, as shown in FIGS. 12A and 12B, it was found that the cytosolic level of NF-κB was reduced in the negative control group compared to the control group, and the nuclear level of NF-κB increased in the negative control group compared to the control group. On the other hand, it was found that, in the paeoniflorin-treated groups (Pae 10 and Pae 20), the cytosolic level of NF-κB increased and the nuclear level of NF-κB decreased.

As shown in FIG. 12C, it was found that the target DNA binding activity of NF-κB, which increased in the negative control group, also decreased in the paeoniflorin-treated groups (Pae 10 and Pae 20).

Thus, these results suggest that NF-κB signaling is activated during cisplatin-induced muscle wasting, and paeoniflorin significantly attenuates cisplatin-induced NF-κB signaling.

### 2-7. Effect of Paeoniflorin on Reduction in Plasma TNF-α

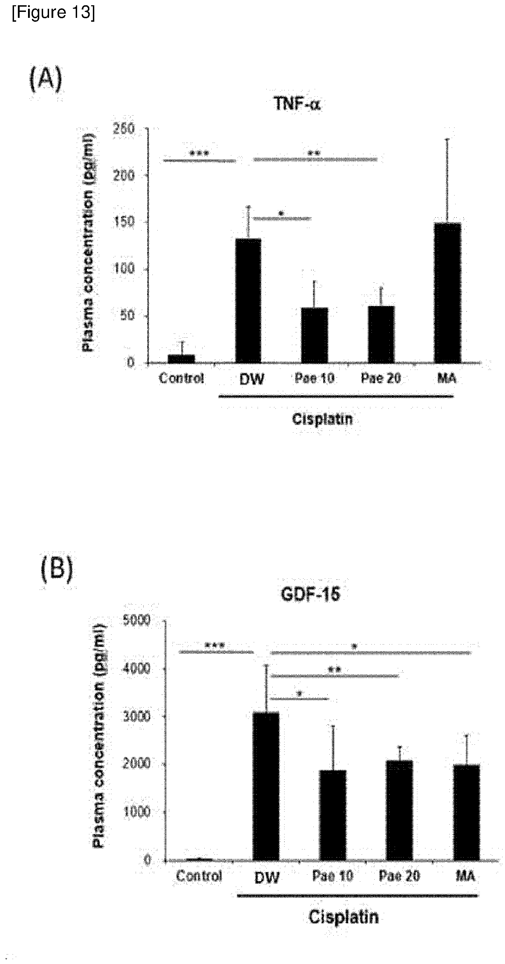

The protein concentration of the pro-inflammatory cytokine TNF-α in the plasma of mice was analyzed by ELISA. As a result, as shown in FIG. 13A, it was found that the plasma concentration of TNF-α significantly increased in the cachexia-induced groups compared to the control group. On the other hand, it was found that the plasma concentration of TNF-α significantly decreased in the cisplatin plus paeoniflorin-treated group compared to the cisplatin-treated group among the cachexia-induced groups.

### 2-8. Effect of Paeoniflorin on Reduction in Plasma GDF-15

The protein concentration of GDF-15 in the plasma was analyzed by ELISA. As a result, as shown in FIG. 13B, it was found that the plasma concentration of GDF-15 significantly increased in the cachexia-induced groups compared to the control group. On the other hand, it was found that the plasma concentration of GDF-15 significantly decreased in the cisplatin plus paeoniflorin-treated group compared to the cisplatin-treated group among the cachexia-induced groups.

### Example 3. Evaluation of Effect on Alleviation of Tumor-Induced Cachexia and Muscle Loss

### 3-1. Experimental Animals and Experimental Methods

As the experimental animals, 4-week-old female C57BL/6 mice and Balb/c mice weighing 18 g to 20 g were purchased from Raon Bio (Yongin, Korea), acclimatized in the laboratory animal facility for one week, and then used in the experiment. During the experiment period, the animals were provided with solid feed and water *ad libitum,* and the breeding room was maintained at a temperature of 20°C to 24°C and a relative humidity of 55% to 65% with a 12-hr light/12-hr dark cycle.

Mice were divided into a control group and a cachexia-induced group, and the cachexia-induced group was subdivided into a tumor cell-injected group, a tumor cell-injected and existing drug (MA, megestrol acetate)-administered group, and tumor cell-injected and paeoniflorin (Pae)-administered groups. Each group consisted of 10 animals.

Each mouse of the control group was injected subcutaneously with 100 µL of saline solution. For the cachexia-induced groups, Lewis lung carcinoma cells (C57BL/6 mice) or colon-26 carcinoma cells (Balb/c mice) were injected subcutaneously into the flank area of each mouse at a dose of 10⁶ cells/100 µL/mouse. After 2 weeks, when a distinct tumor was formed, the tumor cell-injected group was orally administered distilled water, the tumor cell-injected and existing drug-administered group was orally administered megestrol acetate (MA), which is currently most commonly prescribed for cancer cachexia patients in clinical practice, at a dose of 160 mg/kg (= 800 mg [daily human dose]/60kg [average human body weight] × 12 [dose equivalent factor based on human: mouse body surface area ratio], once daily for 9 days, and the tumor cell-injected and paeoniflorin-administered groups were orally administered paeoniflorin (prepared in Example 1) once daily for 9 days at doses of 5 (Pae 5 group), 10 (Pae 10 group) and 20 (Pae 20 group) mg/kg, respectively. After completion of 9 days of administration, the muscle strength of the mice was measured using a grip strength test device (Bioseb, USA), and the endurance of the mice was measured using a treadmill device (Columbus Instrument, USA).

After completion of the experiment, the mice were euthanized, blood was collected therefrom, the body weight excluding tumors, and the weights of the hind limb gastrocnemius muscle and quadriceps muscle were measured. GDF-15 in the serum was quantified by ELISA.

### 3-2. Effect of Paeoniflorin on Improvement of Body Weight and Muscle Mass

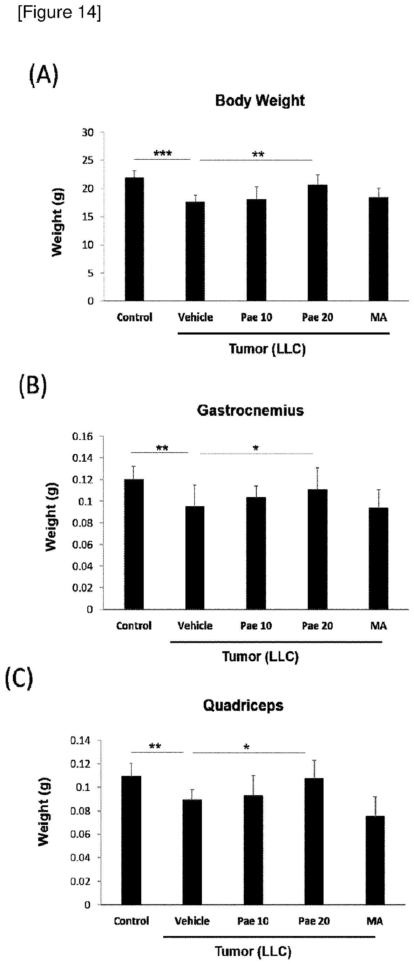

Paeoniflorin was orally administered to the C57BL/6 mice with Lewis lung carcinoma-induced cachexia, and then the weights of the mice were measured. As a result, as shown in FIG. 14A, it could be found that the body weight significantly decreased in the cachexia-induced groups compared to the control group, and the body weight significantly increased in the Lewis lung carcinoma-injected and paeoniflorin (Pae)-administered groups compared to the Lewis lung carcinoma-injected group among the cachexia-induced groups.

After oral administration of paeoniflorin to the C57BL/6 mice with Lewis lung carcinoma-induced cachexia, the mice were euthanized, and the weights of the gastrocnemius and quadriceps muscles among the hind limb muscles were measured. As a result, as shown in FIGS. 14B and 14C, it was found that the weights of the gastrocnemius muscle and quadriceps muscle significantly increased in the Lewis lung carcinoma-injected and paeoniflorin-administered groups compared to the Lewis lung carcinoma-injected group. In this study, megestrol acetate (MA) was used as a comparative drug (positive control), administration of paeoniflorin at a dose of 10 to 20 mg/kg/day showed a better effect than administration of megestrol acetate (MA) at a dose of 160 mg/kg/day.

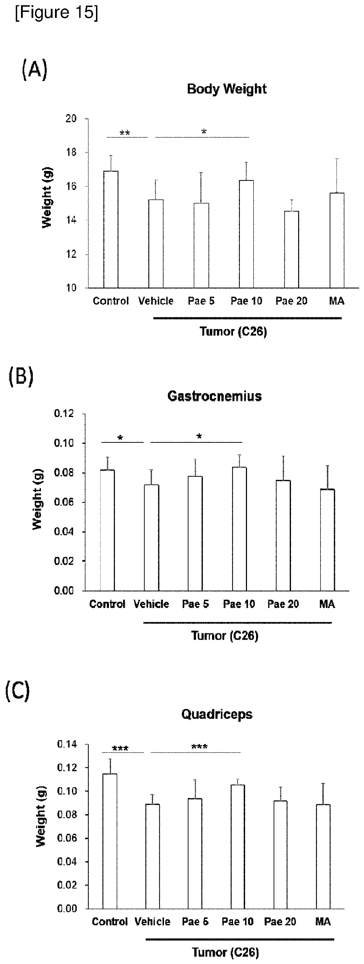

Paeoniflorin was orally administered to the Balb/c mice with colon-26 carcinoma-induced cachexia, and then the weight of the mice was measured. As a result, as shown in FIG. 15A, it could be confirmed that the body weight was significantly reduced in the cachexia-induced groups compared to the control group (control), and the body weight significantly increased in the colon-26 carcinoma-injected and paeoniflorin (Pae)-administered group compared to the colon-26 carcinoma-injected group among the cachexia-induced groups.

After oral administration of paeoniflorin to the Balb/c mice with colon-26 carcinoma-induced cachexia, the mice were euthanized and the weights of the gastrocnemius and quadriceps muscles among the hind limb muscles were measured. As a result, as shown in FIGS. 15B and 15C, it was found that the weights of the gastrocnemius muscle and quadriceps muscle significantly increased in the colon-26 carcinoma-injected and paeoniflorin-administered groups compared to the colon-26 carcinoma-injected group.

### 3-3. Effect of Paeoniflorin on Improvement of Muscle Function

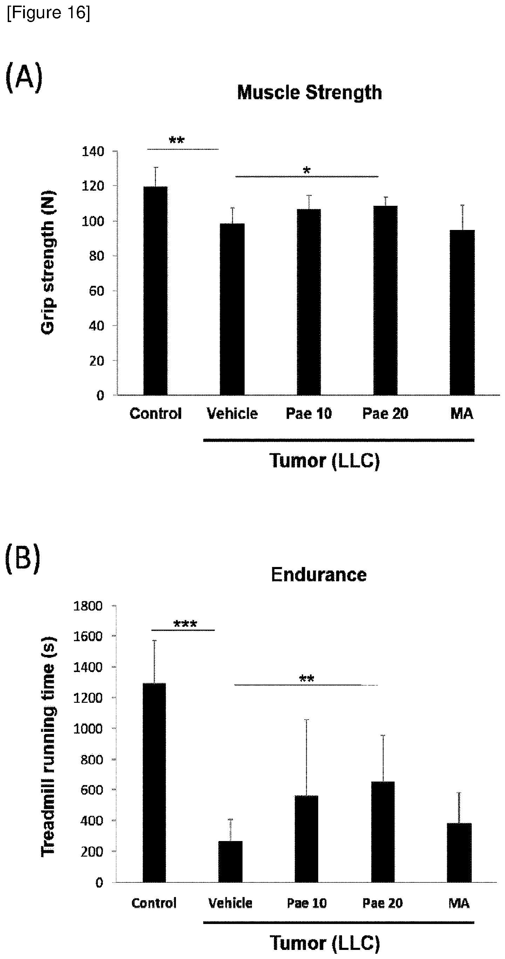

After paeoniflorin was orally administered to the Lewis lung carcinoma-injected C57BL/6 mice for 9 days, muscle function, including muscle strength and endurance, was measured. As a result of the measurement, as shown in FIGS. 16A and 16B, it was found that muscle strength and endurance were significantly reduced in the Lewis lung carcinoma-injected group compared to the control group. On the other hand, it was found that muscle strength and endurance significantly increased in the Lewis lung carcinoma-injected and paeoniflorin-administered groups. In this study, megestrol acetate (MA) was used as a comparative drug (positive control), administration of paeoniflorin at a dose of 10 to 20 mg/kg/day showed a better effect than administration of megestrol acetate (MA) at a dose of 160 mg/kg/day.

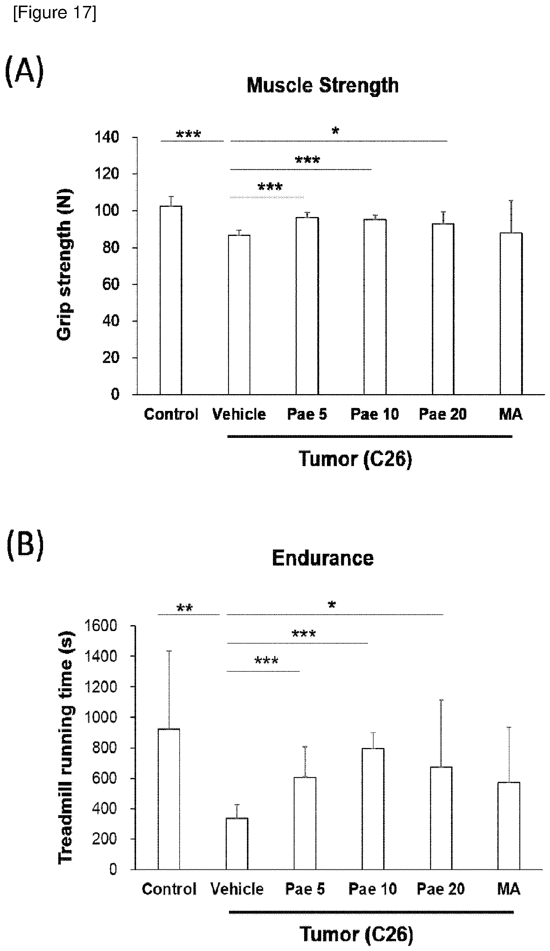

After paeoniflorin was orally administered to the colon-26 carcinoma-injected Balb/c mice for 9 days, muscle function, including muscle strength and endurance, was measured. As a result of the measurement, as shown in FIGS. 17A and 17B, it was found that muscle strength and endurance were significantly reduced in the colon-26 carcinoma-injected group compared to the control group. On the other hand, it was found that muscle strength and endurance significantly increased in the colon-26 carcinoma-injected and paeoniflorin-administered groups.

### 3-4. Effect of Paeoniflorin on Reduction of GDF-15 in Plasma

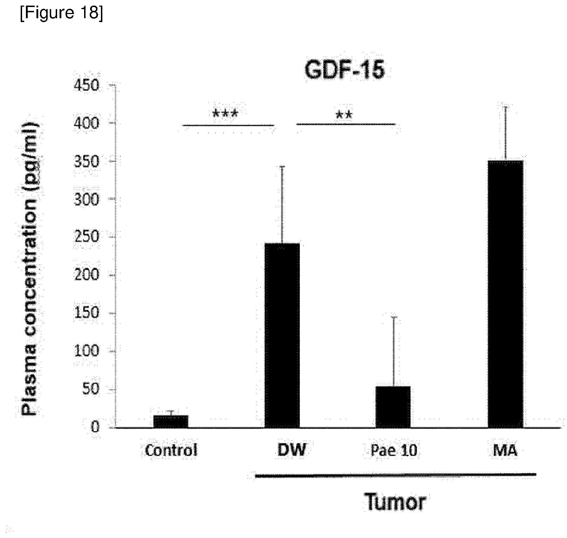

After oral administration of paeoniflorin to the tumor-induced mice for 9 days, blood was collected from the mice, and the plasma concentration of GDF-15 protein was analyzed by ELISA. As a result, as shown in FIG. 18, it was found that the plasma concentration of GDF-15 significantly increased in the Lewis lung carcinoma-injected group compared to the control group. On the other hand, it was found that the serum concentration of GDF-15 significantly decreased in the Lewis lung carcinoma-injected and paeoniflorin-administered group.

### Example 4. Evaluation of Effect on Promotion of Myotube Differentiation of Myoblasts

### 4-1. Cells and Experimental Methods

C2C12 myoblasts were purchased from ATCC (Manassas, Virginia, USA) and cultured using DMEM containing 10% fetal bovine serum. After the cells reached 70% confluence, myotube differentiation was induced while the medium was replaced with DMEM containing 2% horse serum every two days.

A negative control group was treated with 100 ug/ml of TNF-α, which inhibits myotube differentiation. While the myoblasts were treated with paeoniflorin at a concentration of 50 to 200 µM along with TNF-α, the degree of myotube differentiation was observed and the length of differentiated myotubes was measured using the Image J program.

### 4-2. Effect of Paeoniflorin on Promotion of Myotube Differentiation

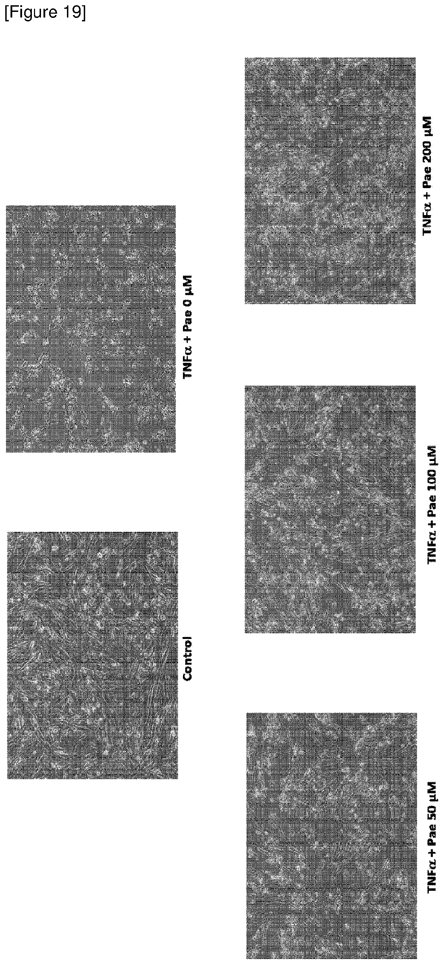

As shown in FIG. 19, it was found that myotube differentiation was clearly suppressed in the negative control group (TNF-α + Pae 0 µM) compared to the control group (control) . On the other hand, it was found that when the myoblasts were treated with paeoniflorin at a concentration of 50 to 200 µM along with TNF-α, myotube differentiation was promoted.

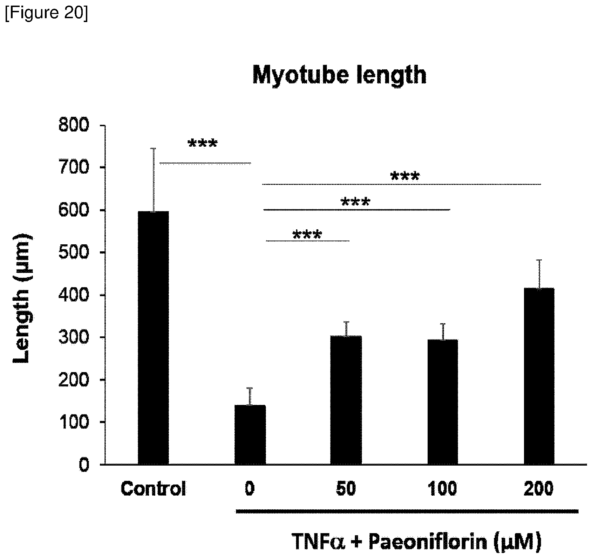

As shown in FIG. 20, it was confirmed that the myotube length significantly increased in the paeoniflorin-treated groups compared to the negative control group (TNF-α + Pae 0 µM). Therefore, it was found that paeoniflorin promoted myotube differentiation.

So far, the present invention has been described with reference to the embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. A pharmaceutical composition for preventing or treating cachexia and muscle loss containing paeoniflorin represented by the following Formula 1:

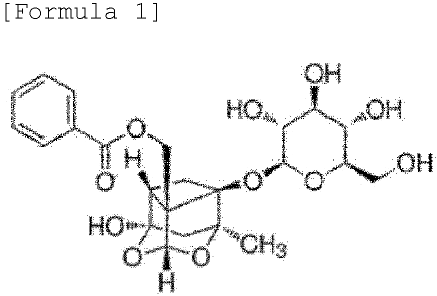

2. The pharmaceutical composition according to claim 1, wherein the cachexia is caused by cancer.

3. The pharmaceutical composition according to claim 1, wherein the cachexia is caused by an anticancer drug.

4. The pharmaceutical composition according to claim 1, wherein the composition alleviates a symptom selected from the group consisting of weight loss, reduced muscle mass, reduced muscle strength, reduced endurance, reduced motor coordination, and increased pro-inflammatory cytokines.

5. The pharmaceutical composition according to any one of claims 1 to 4, wherein the preventing or treating the cachexia and muscle loss is achieved by inhibiting an increase in any one selected from the group consisting of TNF-α, IL-6, IL-1β, and GDF-15.

6. The pharmaceutical composition according to any one of claims 1 to 5, wherein the paeoniflorin is derived from any one plant species selected from the group consisting of *Paeonia suffruticosa* Andrews, *Paeonia lactiflora* Pall., *Paeonia lactiflora var. trichocarpa (Bunge)* Stern, *Paeonia lactiflora f. nuda* Nakai, *Paeonia lactiflora f. pilosella* Nakai, *Paeonia japonica (Makino)* Miyabe & Takeda, *Paeonia japonica var. pillosa* Nakai, *Paeonia obovata* Maxim, *Paeonia veitchii* Lynch, and other related plant species (Paeoniaceae).

7. A food composition for preventing or alleviating cachexia and muscle loss containing paeoniflorin represented by the following Formula 1:

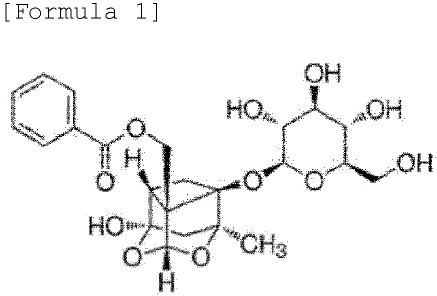

8. An anticancer adjuvant containing paeoniflorin represented by the following Formula 1:
